# EUROPÄISCHE PATENTSCHRIFT

(11) **EP 3 987 282 B1**
(45) Veröffentlichungstag und Bekanntmachung des Hinweises auf die Patenterteilung: **02.12.2026**
(21) Anmeldenummer: 20732730.5
(22) Anmeldetag: 08.06.2020
(51) Int. Cl.: G01N 27/87

(54) **GERÄT ZUR PRÜFUNG VON STAHLDRAHTSEILEN UND VERFAHREN ZU DESSEN ANWENDUNG**
DEVICE FOR TESTING STEEL CABLES, AND METHOD FOR THE USE OF SAID DEVICE
APPAREIL POUR VÉRIFIER DES CÂBLES EN ACIER ET PROCÉDÉ POUR SON APPLICATION

(30) Priorität: 18.06.2019 DE 102019004240
(43) Veröffentlichungstag der Anmeldung: 27.04.2022
(73) Patentinhaber: Pfennig, Mike, 04158 Leipzig (DE); Hofmann, Christian, 04275 Leipzig (DE); Sonntag, Falk, 04347 Leipzig (DE)
(72) Erfinder: SONNTAG, Falk, 04347 Leipzig (DE); HOFMANN, Christian, 04129 Leipzig (DE)
(74) Vertreter: Weidner Stern Jeschke
(86) Internationale Anmeldenummer: PCT/EP2020/025266
(87) Internationale Veröffentlichungsnummer: WO 2020/253984

(56) Entgegenhaltungen:
- EP-A1- 2 581 289
- DE-A1- 19 601 707
- DE-A1- 2 156 434
- GB-A- 2 277 993
- US-A- 4 546 316
- US-A- 5 321 356
- US-A- 5 828 213
- US-A1- 2015 130 454
- US-A1- 2016 325 970
- US-A1- 2017 023 347
- US-A1- 2019 086 239
- US-B2- 9 417 212

## Beschreibung

Die Erfindung betrifft ein Gerät zur Prüfung von Stahldrahtseilen und ein Verfahren zu dessen Anwendung, insbesondere zur Prüfung von hängenden Stahldrahtseilen oder Ketten.

Das Prüfgerät zur zerstörungsfreien Prüfung von Stahldrahtseilen soll zur ortsunabhängigen Prüfung von Stahldrahtseilen mit unterschiedlichem Durchmesser im eingebauten, frei hängenden oder abschnittsweise gespannten Zustand zu verwenden sein.

Tragseile von insbesondere Aufzuganlagen sind sicherheitsrelevante Betriebsmittel und verfügen über eine nur begrenzte Lebensdauer. Aus diesem Grunde ist der Zustand der Seile regelmäßig zu überprüfen.

Eine derartige Überprüfung bei Hebezeugen, insbesondere bei Aufzugsanlagen mit herkömmlichen Drahtseilen, erfolgt regelmäßig mittels Sichtprüfungen durch Sachverständige im Rahmen von wiederkehrenden bzw. regelmäßigen Prüfungen der gesamten Anlage. Üblicherweise werden dabei Drahtbrüche festgestellt und ausgezählt. Weiterhin hat eine stichprobenartige Überprüfung des Seildurchmessers zu erfolgen, welche üblicherweise händisch durchgeführt wird.

Für eine weitergehende Prüfung der montierten Stahldrahtseile musste entweder das Seil ausgebaut werden, um es händisch zu prüfen oder es musste durch eine Person abgefahren werden, um es händisch und äußerlich zu prüfen.

Durch den Ausbau des Seiles entstehen Stillstands- und damit unproduktive Zeiten für die Nutzer von zum Beispiel Arbeitsbühnen.

Beim Abfahren des Seiles müssen entweder die zu prüfenden Seile genutzt werden, deren Zustand man nicht kennt oder es müssen zusätzliche Seile installiert werden, wobei es wieder zu Stillstandszeiten kommt.

Bekannt sind für über äußerliche Prüfungen hinausgehende Prüfungen beispielsweise die Lösung gemäß EP 0 286 712 A2

Diese beschreibt ein Gerät zur Prüfung von ferromagnetischen Stahldrahtseilen, bei dem das zu prüfende Förderseil durch das Feld von Permanentmagneten getrieben wird und Streufelder erfasst werden, wobei wenigstens eine vorzugsweise seilumfassende Induktionsspule vorgesehen ist und der Messeinrichtung eine Auswerteelektronik nachgeschaltet ist, ist erfindungsgemäß vorgesehen, dass zur Erfassung der Streufelder Differentialmessspulen dienen und wenigstens eine weitere Induktionsspule mit nachgeschaltetem Integrator den Seilquerschnitt wiedergebende Signale abgibt, und dass der mit einer opto-elektronischen bzw. elektrischen Abtastung des zu prüfenden Förderseils festgestellte Seildurchmesser, der festgestellte metallische Seilquerschnitt sowie die Summe der Streufelder zusammen mit einer Wegmarke und einer Zeitmarke registriert sind.

In EP 2 383 566 A1 wird ein Verfahren zum computergestützten, optischen Prüfen eines Seils beschrieben, welche umfasst: - Bereitstellen, z.B. mit einer Kamera, eines Bilddatensatzes zu zumindest einem Abschnitt des Seils ; - Bereitstellen von Sollwerten einer bildlichen Längserstreckung der Darstellung von Drähten relativ zu der bildlichen Längserstreckung des Seils in dem Bilddatensatz; - Bestimmen eine bildlichen Längserstreckung der Drähte in dem Bilddatensatz, wobei das Bestimmen ein Anpassen einer angenommenen Längserstreckung an den Bilddatensatz umfasst; - Bestimmen zumindest eines Gütewertes mittels einer Gütenorm als Funktion der bestimmten bildlichen Längserstreckung der Drähte und der Sollwerte der bildlichen Längserstreckung der Drähte; - Diskriminieren von bildlichen Positionen innerhalb des Bilddatensatzes des Seils, an denen zumindest ein Gütewert einen vorbestimmten zugeordneten Gütegrenzwert überschreitet oder unterschreitet; - Bereitstellen der diskriminierten bildlichen Positionen sowie ein zugehöriges System und ein Computerprogrammprodukt.

Ein weiteres Verfahren zur Prüfung von Tragmitteln von Hebezeugen, insbesondere Seilen von Aufzugsanlagen, wird in der EP 1 914 186 A1 mit folgenden Schritten beschrieben: - permanente Bereitstellung einer Auswerteeinheit an einem Hebezeug, - temporäre Bereitstellung einer wenigstens einen Sensor aufweisenden Sensoreinrichtung zur Ermittlung von Sensorsignalen als Grundlage einer Prüfung der Ablegereife der Tragmittel an dem Hebezeug, - In-Wirkverbindung-Bringen der permanent bereitgestellten Auswerteeinheit mit der temporär bereitgestellten Sensoreinrichtung zur Auswertung der ermittelten Sensorsignale zur Bereitstellung einer Information bezüglich der Ablegereife der Tragmittel.

Die Offenlegungsschrift DE 196 01 707 A1 beschreibt ein elektromagnetisches Prüfgerät zum Erfassen eines Metall-Oberflächenverlustes und interner sowie externer Defekte in länglichen bzw. langgestreckten, magnetisch durchlässigen Gegenständen.

Bekannt ist aus dem US- Patent 5,828,213 A eine Vorrichtung zur zerstörungsfreien magnetischen Prüfung und Inspektion von langgestreckten Gegenständen. Das Gerät umgibt konzentrisch ein zu prüfendes längliches Objekt und umfasst Mittel zur Erzeugung eines magnetischen Feldes in einem länglichen Objekt und einen teilweisen eisenhaltigen Rückfluss für den magnetischen Fluss, der von einem Paar von Magnetanordnungen ausgeht.

Auch bekannt ist eine magnetische Prüfvorrichtung zur zerstörungsfreien Prüfung von länglichen Objekten wie Drähten mit großem Durchmesser oder Kabeln aus dem US-Patent 5,321,356 A.

Aufgabe der Erfindung ist es, eine Lösung vorzuschlagen, welche die Nachteile des bekannten Standes der Technik beseitigt und mittels welcher es möglich ist, Stahldrahtseile oder Ketten im angeschlagenen Zustand zu prüfen, ohne dass das Seil durch eine Person abgefahren werden und händisch geprüft werden muss, wobei der zeitliche Aufwand für die Prüfung verringert und ein besserer Nachweis der Prüfung und eine bessere Dokumentation ermöglicht wird.

Eine weitere zusätzliche manuelle Auswertung der Messdaten kann entfallen.

Diese Aufgabe wird gelöst durch das erfindungsgemäße Gerät gemäß Anspruch 1 und das Verfahren zu seiner Anwendung gemäß Anspruch 15, welches anhand der Abbildungen und Ausführungsbeispielen näher erläutert wird.

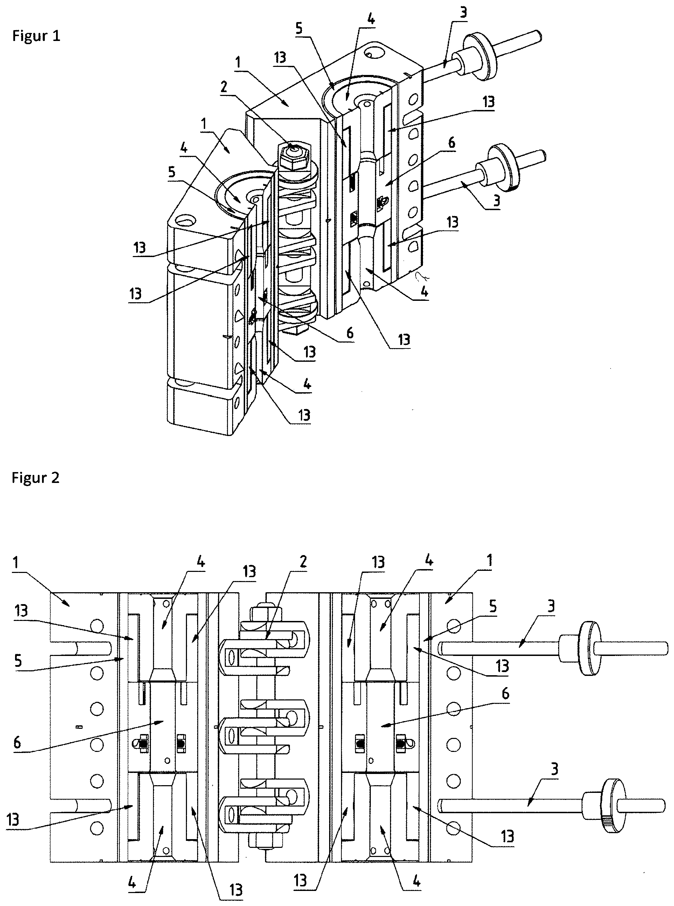

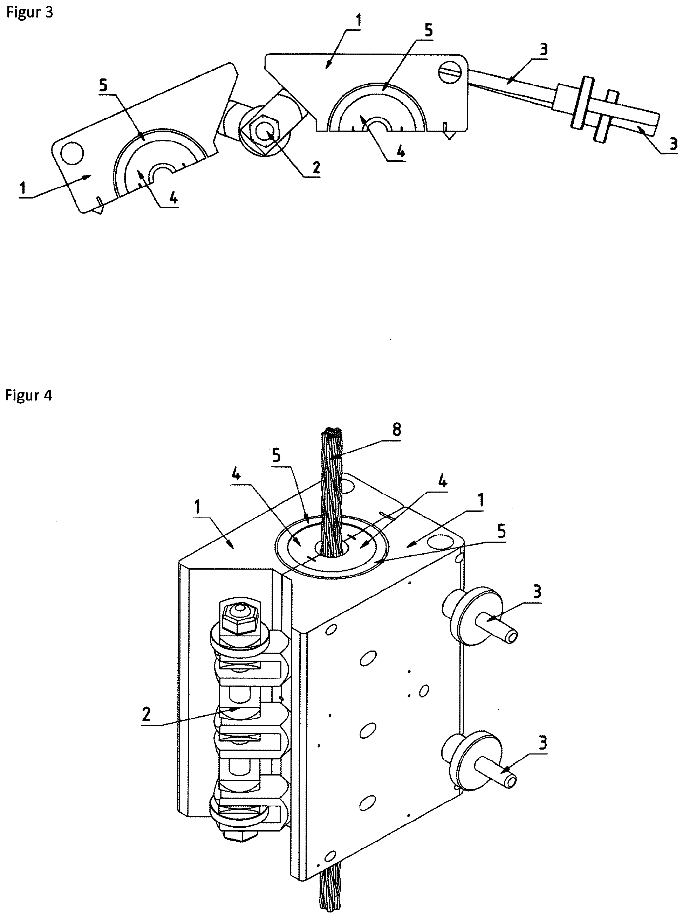

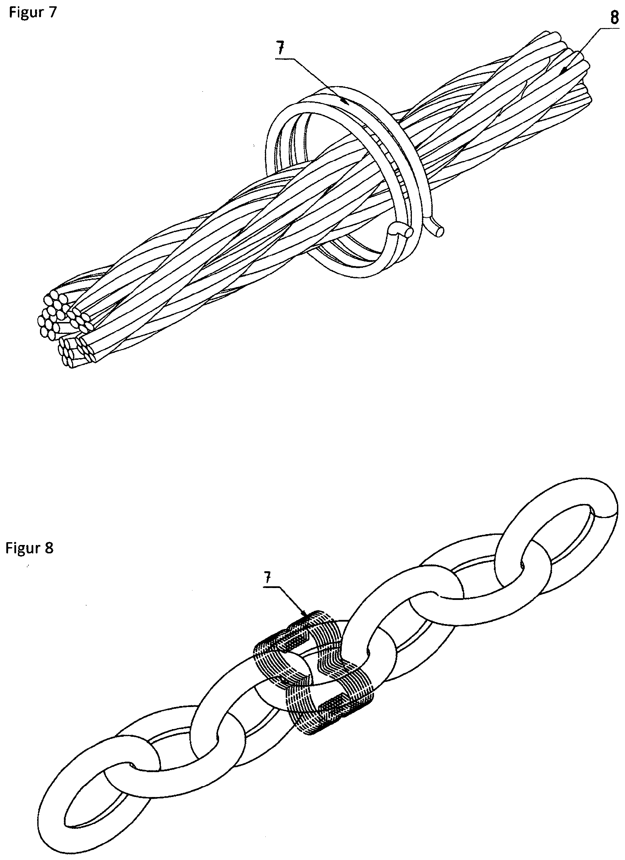

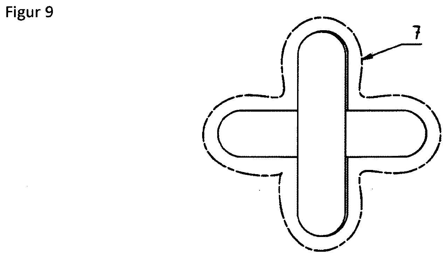

Dabei zeigen die Figuren 1 bis 3 das erfindungsgemäße Gerät in geöffnetem Zustand, Figuren 4 und 5 zeigen das erfindungsgemäße Gerät in geschlossenem Zustand, Figuren 6 bis 9 zeigen Varianten von Sensoren und Figur 10 zeigt einen Signalflussplan.

Die Hauptbestandteile des Gerätes Sensor, Signal- und Datenverarbeitungseinheit 11, Messdatenauswertungseinheit 12 sind modular aufgebaut und können somit für spezielle Anwendungsfälle angepasst und kombiniert werden.

Durch diese vorteilhafte Ausgestaltung ist das erfindungsgemäße Gerät vielfältig einsetzbar, so zum Beispiel bei der Installation an Krananlagen (z.B. Portalkrane) und als direkte Verbindung mit der Kransteuerungseinheit.

Ebenfalls kann das erfindungsgemäße Gerät beispielsweise am Ausgang von Wickelmaschinen zur Herstellung von Stahldrahtseilen zur Qualitätssicherung beim Herstellungsprozess oder zur Überwachung von Seilen oder Ketten von Verlegemaschinen oder Krananlagen installiert werden.

Das Haupteinsatzgebiet des erfindungsgemäßen Gerätes wird jedoch sicherlich die Überprüfung von Seilen und Ketten zur Verwendung im Bereich der Personen- und Lastbeförderung (z.B. Personenaufzüge) sowie bei (Innen-) Befahranlagen für Windkraftanlagen sein.

Das erfindungsgemäße Gerät wird gebildet aus mindestens zwei berührungslos um einen Prüfkörper 8 angeordneten, im geöffneten Zustand äquidistant verbundenen Gehäusesegmenten 1, welche im geschlossenen Zustand radial, zentrumsausgerichteten inneren Flächennormale aufweisen und welche ein tragendes Gehäuse für alle weiteren Bauelemente bilden.

Diese Gehäusesegmente 1 sind an mindestens einer ihrer Längsseiten mittels beweglicher Verbindungsstücke 2 zum Definieren von Abstand, Rotationsachse und Öffnungswinkel der einzelnen Gehäusesegmente 1 miteinander verbunden.

An einem beliebig definierten Längsseitenpaar der Gehäusesegmente 1 ist ein geeigneter Schließmechanismus 3 zur Arretierung des geschlossenen Zustandes und zum Aufbringen der notwendigen Andruckkraft angeordnet.

Des Weiteren weist das erfindungsgemäße Gerät eine mehrteilige, von den Gehäusesegmenten 1 im geschlossenen Zustand zusammengeführte Zentrierungsvorrichtung 4 zum Ausüben der reaktiven Kraft bei Dezentralisierung des Prüfkörpers 8 von der Durchführungsachse der Zentriereinrichtung 4 auf.

Auch weist das erfindungsgemäße Gerät eine mehrteilige, von den Gehäusesegmenten 1 im geschlossenen Zustand zusammengeführte Magnetisierungsvorrichtung 5 zur Erzeugung eines abschnittsweise den Prüfkörper 8 durchflutenden, homogenen und diesen magnetisch sättigenden Magnetfeldes, mit zur Durchführungsachse der Zentrierungeinrichtung 4 kollinearer Längsachse auf.

Innerhalb der Magnetisierungsvorrichtung 5 ist eine Messvorrichtung 6 mit segmentierbaren, automatisch, elektrisch kontaktierbaren, abschnittsweise auf den Umfang des Prüfkörpers 8 bezogenen, äquidistant angeordneten Sensorelementen 7 angeordnet, welche zur Messung der vom Prüfkörper 8 lokal verursachten magnetischen Felder und Übertragung der Messsignale an eine Signal- und Datenverarbeitungseinheit 11 dient.

Weitere Merkmale des erfindungsgemäßen Gerätes sind
- mindestens eine beliebig am Gehäuse angeordnete, mehrteilige Messvorrichtung 6 mit Sensoren für elektromagnetische Wellenemissionen 10 des Prüfkörpers 8 und Übertragung der Messsignale an eine Signal- und Datenverarbeitungseinheit 11
- mindestens ein im Gerät integrierter Wegaufnehmer 9 zur Bestimmung der Länge des Prüfkörpers 8, der Position der Fehlerstellen und ggf. des zurückgelegten Weges des Gerätes, zur Bestimmung der translatorischen Bewegungsparameter des Prüfkörpers 8 wie z.B. relative Geschwindigkeit zwischen Prüfkörper 8 und Gerät und zur Übertragung der Signale an eine Signal- und Datenverarbeitungseinheit 11
- mindestens eine im Gerät integrierte oder kabellose, portable Signalund Datenverarbeitungseinheit 11 zur Aufbereitung der Signale und Erstellung, Verarbeitung, Speicherung und Übertragung der Messwerte
- mindestens eine integrierte oder segregierte, datenübertragungsfähige Messdatenauswertungseinheit 12 zur Mustererkennung, Fehlerklassifizierung, Datenkorrelation zur Erstellung eines Diagnoseberichts und Übertragung aller Daten an ein Endgerät 14
- mindestens eine elektrische Energieversorgung 15 zur Spannungsversorgung der Messvorrichtungen 6, Signal- und Datenverarbeitungseinheit 11 und Messdatenauswertungseinheit 12

Nachfolgend sollen die Elemente des erfindungsgemäßen Gerätes anhand von Ausführungsbeispielen näher erläutert werden

Das Gehäuse besteht aus zwei spiegelsymmetrischen Segmenten 1 mit Führungsnut und Markierungen für die exakte Positionierung der Segmente untereinander. Die Führungsnut kann durch mechanische Positionierungseinrichtungen ergänzt werden.

In einer anderen Ausführungsart kann das Gehäuse aus mehr als zwei Teilen bestehen.

Die Verbindung 2 der spiegelsymmetrischen Gehäusesegmente 1 wird beispielhaft gebildet aus drei Gabelgelenken mit einer gemeinsamen Rotationsachse und Fixierungsmuttern.

Für eine beliebige Anzahl von Gehäusesegmenten 1 können mehrere Verbindungselemente 2 angebracht werden. Die beispielhaft abgebildeten Gabelgelenke können durch andere, starre oder flexible Verbindungselemente 2 ersetzt werden.

Als Schließmechanismus 3 sind im Gehäuse beispielhaft Quernutbolzen mit Gewindestab und Rändelmuttern in doppelter Ausführung eingelassen. Zum Erreichen des benötigten Andruckmomentes können mechanische (z.B. Spannmechaniken, Schraubverschlüsse, Pneumatikspanner), hydraulische (z.B. Hydraulikspanner) oder elektromechanische (z.B. Servo-, BLDC- oder DC-Motor) Schließmechanismen verwendet werden.

Bei der erfindungsgemäßen Lösung wird über Dehnungsmesstreifen, die im Schließmechanismus 3 oder im Gehäuse angebracht sind, die Andruckkraft überwacht.

Die Zentrierungeinrichtung 4 besteht vorzugsweise aus einer rotationssymmetrischen, zweiteiligen Führungshülse in doppelter Ausführung.

Die Zentrierungeinrichtung 4 kann flexibel und variabel ausgeführt werden, um diverse Seilquerschnitte und Seilgeometrien, abhängig von Anzahl der Drähte oder Litzen, Schlag, Wicklungsrichtung, zentrieren zu können. Dafür können die Geometrien der Führungshülsen oder anderweitigen Zentrierungsvorrichtungen 4 je nach Bedarf an die Form des Prüfkörpers 8 angepasst werden. So kann die Führungshülsengeometrie auch zum Zentrieren von Ketten ausgeführt werden.

Die Zentrierungeinrichtung 4 kann durch eine Andruckmechanik, die bei Bedarf in einem Fahrwerk integriert ist, ersetzt werden. Zur Überwachung und Regelung der notwendigen Andruckkraft können im Fahrwerk mechanische Sensoren, beispielsweise Dehnungsmessstreifen oder piezoelektrische Elemente, angebracht werden.

Ebenfalls dem Erfindungsgedanken zugehörig ist eine mehrteilige Ausführung der Zentrierungeinrichtung 4.

In einer weiteren Ausgestaltung kann die Zentrierungeinrichtung 4 berührungslos, mittels elektromagnetischer Regelung ausgeführt werden.

Eine Alternative zum Einsparen der Zentrierungsvorrichtung 4 kann mithilfe der Messdatenauswertungseinheit 12 realisiert werden, da durch diese ein genaues Abbild des Prüfkörpers 8 unabhängig von dessen relativer Position innerhalb des Gerätes erstellt wird.

In einer besonderen Ausführungsform wird die Magnetisierungseinrichtung 5 gebildet aus diametral magnetisierten, äquidistant, rotationssymmetrisch angeordneten Ringsegmentmagneten 13 mit Eisenrückschluss, die zu je acht pro Sensorhälfte mit jeweils vier einen gemeinsamen inneren Nordpol und jeweils vier einen gemeinsamen inneren Südpol bilden.

Im Weiteren weist die Magnetisierungseinrichtung 5 einen ferromagnetischen Eisenrückschluss zur Konzentrierung der magnetischen Feldlinien im äußeren Bereich auf.

Die Ringsegmentmagnete 13 können durch andere Magnetformen und -arten oder durch Elektromagnete ersetzt werden.

Mittels weiterer Sensoren 7 kann eine elektromagnetische Magnetisierungseinrichtung 6 überwacht und geregelt werden, um eine ausreichend hohe und homogene magnetische Durchflutung sicherzustellen.

Die Magnetisierungseinrichtung 5 kann in ihrer Ausführung an verschiedene Prüfkörpergeometrien angepasst werden.

Eine weitere mögliche Ausführung der Magnetisierungseinrichtung 5 wird durch eine Spulenanordnung, wie beispielsweise eine Helmholtzspule realisiert.

Die Messvorrichtung 6 ist vorzugsweise symmetrisch zweiteilig ausgeführt und bildet eine Fassung für äquidistant angeordnete Sensoren 7, beispielsweise Hallsensoren und kann darüber hinaus um den Prüfkörper 8 zu schließende Induktionsschleifensegmente enthalten. Die Messvorrichtung 6 enthält Durchführungen für elektrische (Signal)-Leitungen. Zusätzlich zur Halterung bietet die Messvorrichtung 6 Schutz vor mechanischer Beschädigung der Sensorelemente 7.

Alternativ kann die Messvorrichtung 6 auch außerhalb, aber in unmittelbarer Nähe der Magnetisierungseinrichtung 5 angebracht sein.

Die Positionierung der in der Messvorrichtung 6 enthaltenen Sensorelemente 7 kann durch eine Mechanik automatisch geregelt und die Ausgangssignale können somit an die Prüfkörpergeometrie angepasst werden.

Die Messvorrichtung 6 kann mehrteilig ausgeführt sein.

In einer weitere Ausführungsform der Messeinrichtung 6 werden analoge Sensorsignale in digitale Messwerte umgewandelt und zwischen den Gehäusesegmenten 1 übertragen.

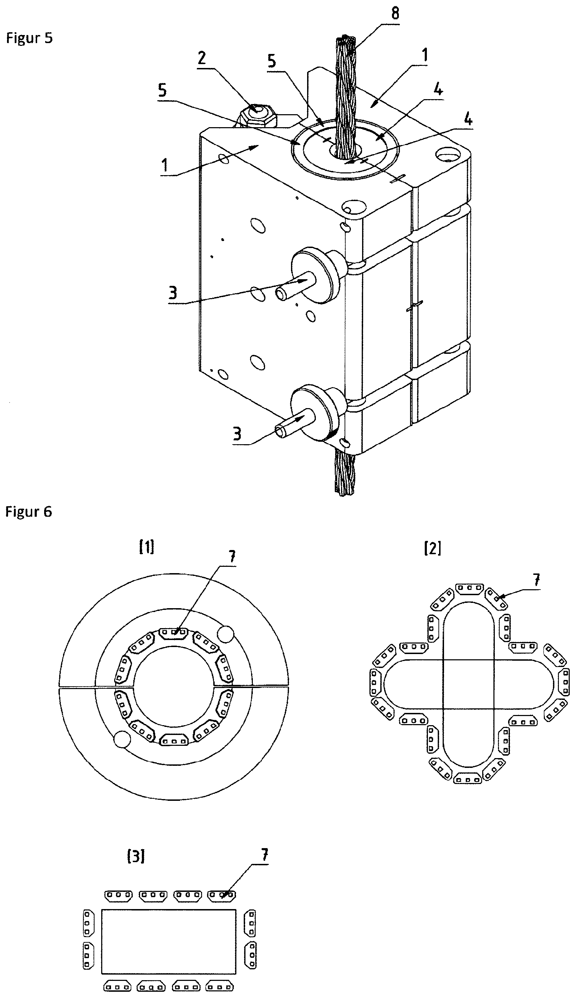

Die Messeinrichtung 6 kann an verschiedene Prüfkörpergeometrien angepasst werden. Ein Beispiel dafür zeigt Fig.6.

Eine weitere vorteilhafte Ausführungsform des Gerätes enthält Infrarotsensoren zur Abtastung des Oberflächenprofils der zu prüfenden Stahldrahtseile oder Ketten, zur besseren Differenzierung verschiedener Fehlerarten, wie z.B. Rost oder Oberflächenverschmutzungen.

Ebenfalls kann ein Wegaufnehmer 9 als Inkrementaldrehgeber oder als optischer Sensor (z.B. Laser) ausgeführt werden.

Eine Signal- und Datenverarbeitungseinheit 11 kann auf die Gehäuseteile 1 aufgeteilt werden oder zentral angeordnet sein. Die Signal- und Datenverarbeitungseinheit 11 kann sämtliche Messdaten simultan in jedem Messzeitpunkt aufnehmen, speichern und übertragen.

Eine Messdatenauswertungseinheit 12 kann über Merkmalsextraktion aus den Messdaten automatisiert Fehler klassifizieren. Die erhobenen Daten werden in eine Datenbank 16 innerhalb oder außerhalb des Gerätes eingepflegt, um eine Rückführbarkeit der Messungen zu gewährleisten und zeitliche Veränderungen des Prüfkörpers 8 zwischen verschiedenen Messungen erkennbar zu machen.

Ebenfalls können vorab Informationen über Sollwerte und spezifische Charakteristika der Prüfkörper 8 in die Datenbank 16 aufgenommen werden, um eine sofortige Mustererkennung und Fehlerklassifizierung zu gewährleisten.

Die ausgewerteten Daten können für weitere Verarbeitungen ausgegeben werden. Dazu können die Messdaten graphisch, bildlich, tabellarisch oder als Messwertdatei an ein visualisierungs- oder speicherfähiges Endgerät 14 übertragen werden. Alternativ kann eine Visualisierungsmöglichkeit (z.B. Display) am Gerät angebracht werden. Die Übertragung der Messdaten an das Endgerät 14 kann mittels drahtloser Datenübertragung (z.B. WLAN) erfolgen.

Die Messdatenauswertungseinheit 12 muss nicht zwingend mit der Signal- und Datenverarbeitungseinheit 11 verbunden sein.

Durch einen in der Messdatenauswertungseinheit 12 implementierten Signalverarbeitungsalgorithmus kann die Zentrierungsvorrichtung 4 entfallen.

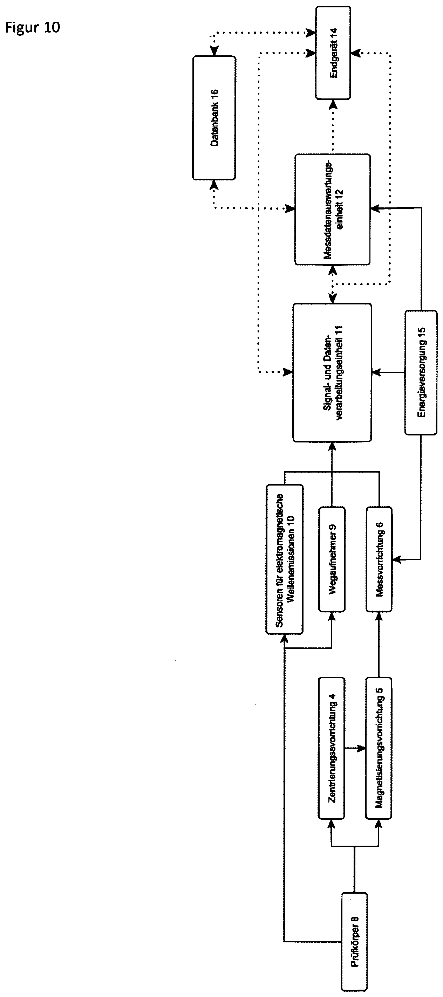

Nachfolgend soll das Verfahren zur Verwendung des erfindungsgemäßen Gerätes anhand des Signalflussplans Fig.10 erläutert werden.
- händisches Heranführen der Zentrierungseinrichtung 4 des erfindungsgemäßen Gerätes an den Prüfkörper 8
- Umschließen des Prüfkörpers 8 und Arretieren der Gehäusesegmente 1 durch Aufbringen der notwendigen Anzugkraft entgegen der inneren Abstoßungskraft mittels Schließvorrichtung 2, Schließen aller elektrischen Kontakte
- abschnittsweise magnetisch sättigende Durchflutung des Prüfkörpers 8 durch die Magnetisierungseinrichtung 5 und Hervorrufen von magnetischen Axial- und Radialfeldern entsprechend des geometrischen Profils und der Permeabilität des Prüfkörpers 8
- Herbeiführen einer relativen Bewegung zwischen Gerät und Prüfkörper 8 entlang der Längsachse der Zentrierungsvorrichtung 4
- Übertragen des magnetischen Profils des Prüfkörpers 8 über die Sensoren 7 der Messvorrichtung 6 in analoge Messsignale
- Automatisches Kalibrieren und Justieren der Signal- und Datenverarbeitungseinheit (11)
- Simultanes Umsetzen und Speichern der analogen Messsignale aller Sensoren 7 in einen digitalen Datenstrom durch die Signal- und Datenverarbeitungseinheit 11
- Echtzeit Fehlerfrüherkennung durch die Signal- und Datenverarbeitungseinheit 11 und Übertragung der Position der Fehlerstelle an vorzugsweise mobiles Endgerät 14
- Zum Messprozess synchrone Berechnung des geometrischen Profils des Prüfkörpers 8 auf Grundlage des magnetischen Profils durch die Messdatenauswertungseinheit 12
- Mustererkennung und Klassifizierung von Fehlerstellen und Erstellen eines Gesamtabbildes durch die Messdatenauswertungseinheit 12
- Automatisches Erstellen einer Diagnose durch Abgleich von Gesamtabbild und Sollparametern des Prüfkörpers 8 durch die Messdatenauswertungseinheit 12
- Übertragen der spezifischen Diagnoseberichte in eine Datenbank 16 zur Feststellung zeitlicher Veränderungen und Erstellung einer Prognose
- Speichern und Ausgabe eines Prüfberichtes an das Endgerät 14

Weitere vorteilhafte Ausgestaltungen der erfindungsgemäßen Lösung werden nicht abschließend gesehen in
- einer möglichen Kombination einer Antriebseinheit für die Seilbefahrung
- einer Anpassung der Bauteilgeometrie von Ringsegmentmagneten 13, Eisenrückschluss, Fassung, Führungshülse für die Messung ähnlicher Prüfkörpergeometrien, wie z.B. Ketten, Seilstrümpfe und unterschiedlicher Querschnitte
- einer Anpassung der Datenverarbeitungseinheit an die Messung ähnlicher Prüfkörpergeometrien, wie z.B. Ketten, Seilstrümpfe etc.
- einer Ergänzung des Gerätes mit Sensoren zur Messung elektromagnetischer Wellenemissionen 10 (z.B. Infrarot) zur besseren Klassifizierung der Fehlerart
- einer Ergänzung des Gerätes mit optischen Sensoren (z.B. Kamera) zur Visualisierung der Fehlstellen
- dem Ersetzen der Quernutbolzen durch elektromechanische Verschlusseinheit
- einer Erweiterung für eine automatische und dynamische Kalibrierung der Messvorrichtung 6 und Signal- und Datenverarbeitungseinheit 11.

Des Weiteren ist möglich, die Initialisierung der Messwertaufnahme mittels Inkrementaldrehgeber des Wegaufnehmers 9 durchzuführen, um eine möglichste hohe Übereinstimmung von Messort und zugehörigem Messwert zu gewährleisten.

Werden in der verwendeten Datenbank 16 hinterlegten Sollwerte z.B. für Durchmesser, Schlag, Draht- / Litzenanzahl, Wicklungsrichtung [rechts/links] erfasst ist eine sofortige Überprüfung der Prüfkörperidentität möglich Ebenfalls ist durch Einsatz optischer Bildgebungsverfahren eine Prüfung weiterer Seilelemente wie z.B. Kauschen möglich.

Als Anwendungsbeispiele für das erfindungsgemäße Gerät und das Verfahren zu seiner Anwendung seien genannt:
Durch die Kombination von Sensor 7 und Signal- und Datenverarbeitungseinheit 11 kann das erfindungsgemäße Gerät fest installiert werden, um eine Permanentüberwachung (Monitoring) der zu überprüfenden Anlage sicherzustellen. Das dafür hinreichende Merkmal des Gerätes ist die Echtzeit-Fehlerfrüherkennung, die durch das Weglassen der weiterführenden Messdatenauswertung auch ohne die Messdatenauswertungseinheit 12 gewährleistet ist.

Konkrete Beispiele dafür sind:
Um das Gerät mobil einzusetzen, kann dieses mit einem Antriebssystem mit vorzugsweise autarker Energiequelle gekoppelt werden.

Dadurch lassen sich folgende Anwendungsbeispiele anführen:
- Die direkte Umgebung des Prüfkörpers 8 kann auf Körper überprüft werden, die den Prüfkörper 8 berühren und somit den Normalbetrieb stören könnten
- Prüfen von Spannsystemen, die bei der Fixierung von hohen Anlagen (z.B. Funktürmen) verwendet werden
- Vorabprüfung von Trag- und Sicherungsseilen bei Außenbefahranlagen (z.B. Rotorblatt- oder Fassadenbefahranlagen)
- Prüfen von Brückenspannseilen (z.B. an Pylonenbrücke)
- Beim Auftreten einer hochgradigen Beschädigung, welche die hinreichenden Ablegekriterien des Prüfkörpers 8 erfüllt, kann das Gerät mit der entsprechenden Diagnose die Prüfung vorzeitig abschließen

Durch die Anpassungsfähigkeit der Messvorrichtung 6, der Magnetisierungsvorrichtung 5 und der Signal- und Datenverarbeitungseinheit 11 an die Prüfkörpergeometrie können beliebige Lastaufnahmemittel (z.B. Hebezeuge, Kettenzüge) geprüft werden, die über ferromagnetische Kraftübertragungselemente verfügen. Hierzu zählen unter anderem beliebige Konfigurationen mit Rundgliederketten, Rollenketten, Stegkette etc.

### Bezugszeichenliste

- 1.: Gehäusesegment
- 2.: Verbindungsstück
- 3.: Schließmechanismus
- 4.: Zentrierungsvorrichtung
- 5.: Magnetisierungsvorrichtung
- 6.: Messvorrichtung
- 7.: Sensorelement
- 8.: Prüfkörper
- 9.: Wegaufnehmer
- 10.: Sensoren für elektromagnetische Wellenemissionen
- 11.: Signal- und Datenverarbeitungseinheit
- 12.: Messdatenauswertungseinheit
- 13.: Ringsegmentmagnet
- 14.: Endgerät
- 15.: Energieversorgung
- 16.: Datenbank

## Patentansprüche

1. Gerät zur Prüfung von Stahldrahtseilen und Stahlketten,
gebildet aus mindestens zwei berührungslos um einen Prüfkörper (8) angeordneten, im geöffneten Zustand äquidistant verbundenen Gehäusesegmenten (1), welche im geschlossenen Zustand radial, zentrumsausgerichtete innere Flächennormalen aufweisen und ein tragendes Gehäuse für alle oder einige weitere Bauelemente bilden und welche an mindestens einer ihrer Längsseiten mittels beweglicher Verbindungsstücke (2) zum Definieren von Abstand, Rotationsachse und Öffnungswinkel der einzelnen Gehäusesegmente (1) miteinander verbunden sind und welche an einem beliebig definierten Längsseitenpaar der Gehäusesegmente (1) einen geeigneten Schließmechanismus (3) zur Arretierung des geschlossenen Zustandes und zum Aufbringen der notwendigen Andruckkraft aufweisen, das Gerät aufweisend eine mehrteilige, von den Gehäusesegmenten (1) im geschlossenen Zustand zusammengeführte Zentrierungsvorrichtung (4) zum Ausüben der reaktiven Kraft bei Dezentralisierung des Prüfkörpers (8) von der Durchführungsachse der Zentrierungsvorrichtung (4), das Gerät aufweisend eine mehrteilige, von den Gehäusesegmenten (1) im geschlossenen Zustand zusammengeführte Magnetisierungsvorrichtung (5) zur Erzeugung eines abschnittsweise den Prüfkörper (8) durchflutenden, homogenen und diesen magnetisch sättigenden Magnetfeldes, mit zur Durchführungsachse der Zentrierungeinrichtung (4) kollinearer Längsachse, wobei innerhalb der Magnetisierungsvorrichtung (5) eine Messvorrichtung (6) mit segmentierbaren, automatisch elektrisch kontaktierbaren, abschnittsweise auf den Umfang des Prüfkörpers (8) bezogenen, äquidistant angeordneten Sensorelementen (7) angeordnet ist, welche zur Messung der vom Prüfkörper (8) lokal verursachten magnetischen Felder und Übertragung der Messsignale an eine Signal- und Datenverarbeitungseinheit (11) dient, und mindestens eine beliebig am Gehäuse angeordnete, mehrteilige Messvorrichtung (6) mit Sensoren für elektromagnetische Wellenemissionen (10) des Prüfkörpers (8) und Übertragung der Messsignale an eine Signal- und Datenverarbeitungseinheit (11) angeordnet ist und mindestens ein im Gerät integrierter Wegaufnehmer (9) zur Bestimmung der Länge des Prüfkörpers (8), der Position der Fehlerstellen und ggf. des zurückgelegten Weges des Gerätes, zur Bestimmung der translatorischen Bewegungsparameter des Prüfkörpers (8) wie z.B. relative Geschwindigkeit zwischen Prüfkörper (8) und Gerät und zur Übertragung der Signale an eine Signal- und Datenverarbeitungseinheit (11) angeordnet ist, mindestens eine im Gerät integrierte oder kabellose, portable Signal- und Datenverarbeitungseinheit (11) zur Aufbereitung der Signale und Erstellung, Verarbeitung, Speicherung und Übertragung der Messwerte angeordnet ist, mindestens eine integrierte oder segregierte, datenübertragungsfähige Messdatenauswertungseinheit (12) zur Mustererkennung, Fehlerklassifizierung, Datenkorrelation zur Erstellung eines Diagnoseberichts und Übertragung aller Daten an ein Endgerät (14) angeordnet ist, und mindestens eine elektrische Energieversorgung (15) zur Spannungsversorgung der Messvorrichtungen (6), Signal- und Datenverarbeitungseinheit (11) und Messdatenauswertungseinheit (12) angeordnet ist, und Dehnungsmesstreifen, die im Schließmechanismus (3) oder im Gehäuse angebracht sind, und eingerichtet sind, die Andruckkraft zu überwachen.

2. Gerät nach Anspruch 1, bei welchem die Zentrierungsvorrichtung (4) aus einer rotationssymmetrischen, zweiteiligen Führungshülse in doppelter Ausführung gebildet ist.

3. Gerät nach Anspruch 1, bei welchem die Zentrierungsvorrichtung (4) flexibel und variabel ausgeführt ist und eingericht ist, die Geometrien der Führungshülsen oder anderweitigen Zentrierungsvorrichtungen (4) je nach Bedarf der Geometrie an die Form des Prüfkörpers (8) anzupassen**.**

4. Gerät nach Anspruch 1, welches ausgestaltet ist, mittels Sensoren (7) die elektromagnetische Magnetisierungseinrichtung (5) zur Sicherstellung einer hohen und homogenen magnetischen Durchflutung zu überwachen und zu regeln.

5. Gerät nach Anspruch 1, welches ausgestaltet ist, die Magnetisierungseinrichtung (5) in ihrer geometrischen Ausführung den jeweiligen Prüfkörpergeometrien anzupassen.

6. Gerät nach Anspruch 1, welches ausgestaltet ist, die Positionierung der in der Messvorrichtung (6) enthaltenen Sensorelemente (7) durch eine Mechanik zu regeln und die Ausgangssignale an die Prüfkörpergeometrie anzupassen**.**

7. Gerät nach Anspruch 1, welches ausgestaltet ist, die Messvorrichtung (6) an verschiedene Prüfkörpergeometrien anzupassen**.**

8. Gerät nach Anspruch 1, bei welchem das Gerät Infrarotsensoren zur Abtastung des Oberflächenprofils der zu prüfenden Stahldrahtseile oder Ketten, zur besseren Differenzierung verschiedener Fehlerarten, wie z.B. Rost oder Oberflächenverschmutzungen aufweist.

9. Gerät nach Anspruch 1, bei welchem ein Wegaufnehmer (9) ) als Inkrementaldrehgeber oder als optischer Sensor ausgeführt ist.

10. Gerät nach Anspruch 1, welches mit einer Antriebseinheit für die Seilbefahrung ausgestattet ist.

11. Gerät nach Anspruch 1, bei welchem die Bauform von Ringsegmentmagneten (13), Eisenrückschluss, Fassung und Führungshülse für die Messung ähnlicher Prüfkörpergeometrien der Geometrie von Ketten, Seilstrümpfen und unterschiedlichen Querschnitten entspricht.

12. Gerät nach Anspruch 1, welches ausgestaltet ist, die Konfiguration der Signal- und Datenverarbeitungseinheit (11) für die Messung ähnlicher Prüfkörpergeometrien, wie z.B. Ketten, Seilstrümpfen etc. deren Geometrie anzupassen**.**

13. Gerät nach Anspruch 1, welches mit Sensoren zur Messung elektromagnetischer Wellenemissionen (10), z.B. Infrarot, zur besseren Klassifizierung der Fehlerart ausgestattet ist.

14. Gerät nach Anspruch 1, welches mit optischen Sensoren, z.B. Kamera, zur Visualisierung der Fehlstellen ausgestattet ist.

15. Verfahren zur Prüfung von Stahldrahtseilen und Stahlketten, mit den Verfahrensschritten
- händisches Heranführen der Zentrierungseinrichtung (4) des Gerätes gemäss Anspruch 1 an den Prüfkörper (8)
- Umschließen des Prüfkörpers (8) und Arretieren der Gehäusesegmente (1) durch Aufbringen der notwendigen Anzugkraft entgegen der inneren Abstoßungskraft mittels Schließvorrichtung (2), Schließen aller elektrischen Kontakte
- abschnittsweise magnetisch sättigende Durchflutung des Prüfkörpers (8) durch die Magnetisierungseinrichtung (5) und Hervorrufen von magnetischen Axial- und Radialfeldern entsprechend des geometrischen Profils und der Permeabilität des Prüfkörpers (8)
- Herbeiführen einer relativen Bewegung zwischen Gerät und Prüfkörper (8) entlang der Längsachse der Zentrierungsvorrichtung (4)
- Übertragen des magnetischen Profils des Prüfkörpers (8) über die Sensoren (7) der Messvorrichtung (6) in analoge Messsignale
- automatisches Kalibrieren und Justieren der Signal- und Datenverarbeitungseinheit (11)
- simultanes Umsetzen und Speichern der analogen Messsignale aller Sensoren (7) in einen digitalen Datenstrom durch die Signal- und Datenverarbeitungseinheit (11)
- Echtzeit Fehlerfrüherkennung durch die Signal- und Datenverarbeitungseinheit (11) und Übertragung der Position der Fehlstelle an ein vorzugsweise mobiles Endgerät (14)
- zum Messprozess vorzugsweise synchrone Berechnung des geometrischen Profils des Prüfkörpers (8) auf Grundlage des magnetischen Profils durch die Messdatenauswertungseinheit (12)
- Mustererkennung und Klassifizierung von Fehlstellen und Erstellen eines Gesamtabbildes durch die Messdatenauswertungseinheit (12)
- Automatisches Erstellen einer Diagnose durch Abgleich von Gesamtabbild und Sollparametern des Prüfkörpers (8) durch die Messdatenauswertungseinheit (12)
- Übertragen der spezifischen Diagnoseberichte in eine Datenbank (16) zur Feststellung zeitlicher Veränderungen und Erstellung einer Prognose
- Speichern und Ausgabe eines Prüfberichtes an das Endgerät (14)

16. Verwendung des Gerätes nach Anspruch **1,** wobei eine Messdatenauswertungseinheit (12) über Merkmalsextraktion aus den Messdaten automatisiert Fehler klassifiziert wird und die erhobenen Daten in eine Datenbank (16) innerhalb oder außerhalb des Gerätes eingepflegt werden, um eine Rückführbarkeit der Messungen zu gewährleisten und zeitliche Veränderungen des Prüfkörpers (8) zwischen verschiedenen Messungen erkennbar zu machen.

17. Verwendung des Gerätes nach Anspruch **1,** bei welchem vorab Informationen über Sollwerte und spezifische Charakteristika der Prüfkörper (8) in die Datenbank (16) aufgenommen werden, um eine sofortige Mustererkennung und Fehlerklassifizierung zu gewährleisten.

18. Verwendung des Gerätes nach Anspruch **1,** bei welchem die ausgewerteten Daten für weitere Verarbeitungen ausgegeben werden, um die Messdaten graphisch, bildlich, tabellarisch oder als Messwertdatei an ein visualisierungs- oder speicherfähiges Endgerät (14) zu übertragen.

19. Verwendung des Gerätes nach Anspruch **1,** bei welchem unter Anordnung einer Visualisierungsmöglichkeit, z.B. Display, am Gerät die Steuerung des Gerätes sowie die Übertragung der Messdaten an das Endgerät (14) mittels drahtloser Datenübertragung, z.B. WLAN, durchzuführen.

20. Verwendung des Gerätes nach Anspruch **1,** bei welchem die Initialisierung der Messwertaufnahme mittels Inkrementaldrehgeber des Wegaufnehmers (9) durchgeführt wird, um eine möglichste hohe Übereinstimmung von Messort und zugehörigem Messwert zu gewährleisten.

21. Verwendung des Gerätes nach Anspruch **1,** bei welchem in der verwendeten Datenbank (16) hinterlegte Sollwerte z.B. für Durchmesser, Schlag, Draht- / Litzenanzahl, Wicklungsrichtung erfasst sind um eine sofortige Überprüfung der Prüfkörperidentität durchzuführen.

22. Verwendung des Gerätes nach Anspruch **1,** um durch Einsatz optischer Bildgebungsverfahren eine Prüfung weiterer Seilelemente wie z.B. Kauschen zu realisieren ist.

## Claims

1. Device for testing steel wire ropes and steel chains,
comprising at least two housing segments (1) arranged contactlessly around a test object (8), which in the open state are connected equidistantly and which in the closed state exhibit radially, centrally aligned inner surface normals and form a supporting housing for all or some further components, and which are connected to one another on at least one of their longitudinal sides by means of movable connecting elements (2) for defining the spacing, axis of rotation, and opening angle of the individual housing segments (1), and which on any arbitrarily defined pair of longitudinal sides of the housing segments (1) comprise a suitable closing mechanism (3) for locking the closed state and for applying the required contact pressure, the device comprising a multipart centering device (4), brought together by the housing segments (1) in the closed state, for exerting the reactive force in the event of decentralization of the test object (8) from the pass-through axis of the centering device (4), the device comprising a multipart magnetization device (5), brought together by the housing segments (1) in the closed state, for generating a homogeneous magnetic field that flows sectionally through the test object (8) and magnetically saturates it, with a longitudinal axis collinear with the pass-through axis of the centering device (4), wherein within the magnetization device (5) a measuring device (6) is arranged having segmentable sensor elements (7) that can be automatically electrically contacted, are arranged equidistantly and sectionally with reference to the circumference of the test object (8), which serve to measure magnetic fields locally caused by the test object (8) and to transmit the measurement signals to a signal and data processing unit (11), and at least one multipart measuring device (6), arranged arbitrarily on the housing, with sensors for electromagnetic wave emissions (10) of the test object (8) and for transmitting the measurement signals to a signal and data processing unit (11) is arranged, and at least one path transducer (9) integrated in the device for determining the length of the test object (8), the position of defect locations and, if applicable, the distance traveled by the device, for determining translational motion parameters of the test object (8), such as the relative velocity between the test object (8) and the device, and for transmitting the signals to a signal and data processing unit (11) is arranged, and at least one integrated or wireless, portable signal and data processing unit (11) for conditioning the signals and for creating, processing, storing, and transmitting the measured values is arranged, and at least one integrated or segregated, data-transmission-capable measurement data evaluation unit (12) for pattern recognition, defect classification, data correlation for generating a diagnostic report, and for transmitting all data to a terminal device (14) is arranged, and at least one electrical power supply (15) for supplying voltage to the measuring devices (6), the signal and data processing unit (11), and the measurement data evaluation unit (12) is arranged, and strain gauges are provided, which are mounted in the closing mechanism (3) or in the housing and are configured to monitor the contact pressure.

2. Device according to claim 1, wherein the centering device (4) is formed by a rotationally symmetrical, two-part guide sleeve in duplicate configuration.

3. Device according to claim 1, wherein the centering device (4) is designed to be flexible and variable and is configured to adapt the geometries of the guide sleeves or other centering devices (4) as required to the geometry of the test object (8).

4. Device according to claim 1, which is configured to monitor and regulate the electromagnetic magnetization device (5) by means of sensors (7) to ensure a high and homogeneous magnetic flux.

5. Device according to claim 2, which is configured to adapt the geometric design of the magnetization device (5) to the respective test object geometries.

6. Device according to claim 1, which is configured to regulate the positioning of the sensor elements (7) contained in the measuring device by means of a mechanical system and to adapt the output signals to the test object geometry.

7. Device according to claim 1, which is configured to adapt the measuring device (6) to different test object geometries.

8. Device according to claim 1, wherein the device comprises infrared sensors for scanning the surface profile of the steel wire ropes or chains to be tested, for improved differentiation of various defect types, such as rust or surface contamination.

9. Device according to claim 1, wherein a path transducer (9) is designed as an incremental rotary encoder or as an optical sensor.

10. Device according to claim 1, which is equipped with a drive unit for traveling along the rope.

11. Device according to claim 1, wherein the structural design of ring-segment magnets (13), magnetic return path, mount, and guide sleeve corresponds to the geometry of chains, rope sockets, and different cross sections for the measurement of similar test object geometries.

12. Device according to claim 1, which is configured to adapt the configuration of the signal and data processing unit (11) to the geometry of similar test object geometries, such as chains, rope sockets, etc.

13. Device according to claim 1, which is equipped with sensors for measuring electromagnetic wave emissions (10), for example infrared radiation, for improved classification of the type of defect.

14. Device according to claim 1, which is equipped with optical sensors, for example a camera, for visualizing defect locations.

15. Method for testing steel wire ropes and steel chains, comprising the method steps:
- manually guiding the centering device (4) of the device according to claim 1 to the test object (8),
- enclosing the test object (8) and locking the housing segments (1) by applying the necessary clamping force against the internal repulsive force by means of the closing device (2), closing all electrical contacts,
- sectionally magnetically saturating fluxing of the test object (8) by the magnetization device (5) and inducing magnetic axial and radial fields corresponding to the geometric profile and permeability of the test object (8),
- producing a relative movement between the device and the test object (8) along the longitudinal axis of the centering device (4),
- transmitting the magnetic profile of the test object (8) via the sensors of the measuring device (6) into analog measurement signals,
- automatic calibration and adjustment of the signal and data processing unit (11),
- simultaneous conversion and storage of the analog measurement signals of all sensors (7) into a digital data stream by the signal and data processing unit (11),
- real-time early defect detection by the signal and data processing unit (11) and transmission of the position of the defect location to a preferably mobile terminal device (14),
- preferably synchronous calculation of the geometric profile of the test object (8) during the measurement process on the basis of the magnetic profile by the measurement data evaluation unit (12),
- pattern recognition and classification of defect locations and creation of an overall representation by the measurement data evaluation unit (12),
- automatic generation of a diagnosis by comparing the overall representation with target parameters of the test object (8) by the measurement data evaluation unit (12),
- transmitting the specific diagnostic reports to a database (16) for determining temporal changes and generating a prognosis,
- storing and outputting a test report to the terminal device (14).

16. Use of the device according to claim 1, wherein a measurement data evaluation unit (12) automatically classifies defects by feature extraction from the measurement data and the collected data are entered into a database (16) inside or outside the device in order to ensure traceability of the measurements and to make temporal changes of the test object (8) between different measurements detectable.

17. Use of the device according to claim 1, wherein information on target values and specific characteristics of the test objects (8) is entered into the database (16) in advance in order to ensure immediate pattern recognition and defect classification.

18. Use of the device according to claim 1, wherein the evaluated data are output for further processing in order to transmit the measurement data graphically, pictorially, in tabular form, or as a measurement data file to a visualization- or storage-capable terminal device (14).

19. Use of the device according to claim 1, wherein, with the arrangement of a visualization option, for example a display, on the device, the control of the device as well as the transmission of the measurement data to the terminal device (14) is carried out by means of wireless data transmission, for example WLAN.

20. Use of the device according to claim 1, wherein the initialization of the measurement value acquisition is carried out by means of the incremental rotary encoder of the path transducer (9) in order to ensure the highest possible correspondence between the measurement location and the associated measured value.

21. Use of the device according to claim 1, wherein target values stored in the database (16), for example for diameter, lay length, number of wires/strands, and winding direction, are recorded in order to carry out an immediate verification of the identity of the test object.

22. Use of the device according to claim 1, in order to realize testing of further rope elements, such as thimbles, by using optical imaging methods.

## Revendications

1. Dispositif pour le contröôle de câäbles métalliques et de chaînes en acier,
comprenant au moins deux segments de boîtier (1) disposés sans contact autour d'un objet à contrôler (8), qui, à l'état ouvert, sont reliés entre eux à distance égale et qui, à l'état fermé, présentent des normales de surface intérieure alignées radialement et centralement et forment un boîtier porteur pour tout ou partie des autres composants, et qui sont reliés entre eux sur au moins l'un de leurs côtés longitudinaux au moyen d'éléments de liaison mobiles (2) destinés à définir l'écartement, l'axe de rotation et l'angle d'ouverture des différents segments de boîtier (1), et qui comportent, sur toute paire arbitrairement définie de côtés longitudinaux des segments de boîtier (1), un mécanisme de fermeture approprié (3) destiné au verrouillage de l'état fermé et à l'application de la pression de contact requise, le dispositif comprenant un dispositif de centrage multipartite (4), réuni par les segments de boîtier (1) à l'état fermé, pour exercer la force de réaction en cas de décentralisation de l'objet à contrôler (8) par rapport à l'axe de passage du dispositif de centrage (4),Ie dispositif comprenant un dispositif de magnétisation multipartite (5), réuni par les segments de boîtier (1) à l'état fermé, pour générer un champ magnétique homogène traversant l'objet à contrôler (8) par sections et le saturant magnétiquement, avec un axe longitudinal colinéaire à l'axe de passage du dispositif de centrage (4),dans lequel, à l'intérieur du dispositif de magnétisation (5), est disposé un dispositif de mesure (6) comportant des éléments capteurs segmentables (7), pouvant être automatiquement contactés électriquement, disposés de manière équidistante et par sections par rapport à la circonférence de l'objet à contrôler (8), lesquels servent à mesurer localement des champs magnétiques provoqués par l'objet à contrôler (8) et à transmettre les signaux de mesure àä une unitée de traitement des signaux et des données (11), et dans lequel est disposé au moins un dispositif de mesure multipartite (6), agencé de manière arbitraire sur le boîtier, avec des capteurs destinés aux émissions d'ondes électromagnétiques (10) de l'objet à contrôler (8) et à la transmission des signaux de mesure à une unité de traitement des signaux et des données (11), et dans lequel est disposé au moins un capteur de déplacement (9) intégré au dispositif pour déterminer la longueur de l'objet à contrôler (8), la position des emplacements de défauts et, le cas échéant, la distance parcourue par le dispositif, pour déterminer des paramètres de mouvement translationnel de l'objet à contrôler (8), tels que la vitesse relative entre l'objet à contrôler (8) et le dispositif, et pour transmettre les signaux à une unité de traitement des signaux et des donnéees (11), et dans lequel est disposée au moins une unité de traitement des signaux et des donnéees (11), intéegréee ou sans fil et portable, destinée au conditionnement des signaux ainsi qu'à la création, au traitement, au stockage et à la transmission des valeurs mesurées, et dans lequel est disposée au moins une unité d'évaluation des données de mesure (12), intégrée ou séparée et apte à la transmission de données, destinée à la reconnaissance de formes, à la classification des défauts, à la corrélation des données pour la génération d'un rapport de diagnostic et à la transmission de toutes les données à un dispositif terminal (14), et dans lequel est disposée au moins une alimentation électrique (15) destinée à fournir une tension aux dispositifs de mesure (6), à l'unité de traitement des signaux et des données (11) et à l'unité d'évaluation des données de mesure (12),
et dans lequel sont prévus des jauges de contrainte, montées dans le mécanisme de fermeture (3) ou dans le boîtier, et configurées pour surveiller la pression de contact.

2. Dispositif selon la revendication 1, dans lequel le dispositif de centrage (4) est formé par une douille de guidage à symétrie de révolution, en deux parties, en configuration double.

3. Dispositif selon la revendication 1, dans lequel le dispositif de centrage (4) est conçu de manière flexible et variable et est configuré pour adapter, selon les besoins, les géométries des douilles de guidage ou d'autres dispositifs de centrage (4) à la géométrie de l'objet à contrôler (8).

4. Dispositif selon la revendication 1, configuré pour surveiller et réguler le dispositif de magnétisation électromagnétique (5) au moyen de capteurs (7) afin de garantir un flux magnétique élevé et homogène.

5. Dispositif selon la revendication 2, configuré pour adapter la conception géométrique du dispositif de magnétisation (5) aux géométries respectives des objets à contrôler.

6. Dispositif selon la revendication 1, configuré pour réguler le positionnement des éléments capteurs (7) contenus dans le dispositif de mesure au moyen d'un système mécanique et pour adapter les signaux de sortie à la géométrie de l'objet à contrôler.

7. Dispositif selon la revendication 1, configuré pour adapter le dispositif de mesure (6) à différentes géométries d'objets à contrôler.

8. Dispositif selon la revendication 1, dans lequel le dispositif comprend des capteurs infrarouges destinés à balayer le profil de surface des câbles métalliques ou des chaînes en acier à contrôler, afin d'améliorer la différenciation de différents types de défauts, tels que la rouille ou les contaminations de surface.

9. Dispositif selon la revendication 1, dans lequel un capteur de déplacement (9) est conçu sous la forme d'un codeur rotatif incrémental ou d'un capteur optique.

10. Dispositif selon la revendication 1, équipé d'une unité d'entraînement destinée au déplacement le long du câble.

11. Dispositif selon la revendication 1, dans lequel la conception structurelle des aimants en segments annulaires (13), du circuit magnétique de retour, du support et de la douille de guidage correspond à la géométrie des chaînes, des manchons de câble et de différentes sections transversales pour la mesure de géométries similaires d'objets à contrôler.

12. Dispositif selon la revendication 1, configuré pour adapter la configuration de l'unité de traitement des signaux et des données (11) à la géométrie de géométries similaires d'objets à contrôler, telles que des chaînes, des manchons de câble, etc.

13. Dispositif selon la revendication 1, équipé de capteurs destinés à mesurer des émissions d'ondes électromagnétiques (10), par exemple un rayonnement infrarouge, afin d'améliorer la classification du type de défaut.

14. Dispositif selon la revendication 1, équipé de capteurs optiques, par exemple une caméra, destinés à visualiser les emplacements de défauts.

15. Procédé de contrôle de câbles métalliques et de chaînes en acier, comprenant les étapes de procédé suivantes :
- guidage manuel du dispositif de centrage (4) du dispositif selon la revendication 1 vers l'objet à contrôler (8),
- enveloppement de l'objet à contrôler (8) et verrouillage des segments de boîtier (1) par application de la force de serrage nécessaire contre la force répulsive interne au moyen du dispositif de fermeture (2), fermeture de tous les contacts électriques,
- saturation magnétique sectionnelle de l'objet à contrôler (8) par le dispositif de magnétisation (5) et induction de champs magnétiques axiaux et radiaux correspondant au profil géométrique et à la perméabilité de l'objet à contrôler (8),
- production d'un mouvement relatif entre le dispositif et l'objet à contrôler (8) le long de l'axe longitudinal du dispositif de centrage (4),
- transmission du profil magnétique de l'objet à contrôler (8) via les capteurs du dispositif de mesure (6) sous forme de signaux de mesure analogiques,
- étalonnage et réglage automatiques de l'unité de traitement des signaux et des données (11),
- conversion et stockage simultanés des signaux de mesure analogiques de tous les capteurs (7) en un flux de données numérique par l'unité de traitement des signaux et des données (11),
- détection précoce de défauts en temps réel par l'unité de traitement des signaux et des données (11) et transmission de la position de l'emplacement du déefaut àä un dispositif terminal (14) de préférence mobile,
- calcul de préférence synchrone du profil géométrique de l'objet à contrôler (8) pendant le processus de mesure sur la base du profil magnétique par l'unité d'évaluation des données de mesure (12),
- reconnaissance de formes et classification des emplacements de défauts et création d'une représentation globale par l'unité d'évaluation des données de mesure (12),
- génération automatique d'un diagnostic par comparaison de la représentation globale avec des paramètres cibles de l'objet à contrôler (8) par l'unité d'évaluation des données de mesure (12),
- transmission des rapports de diagnostic spécifiques à une base de données (16) pour la détermination des évolutions temporelles et la génération d'un pronostic,
- stockage et sortie d'un rapport d'essai vers le dispositif terminal (14).

16. Utilisation du dispositif selon la revendication 1, dans laquelle une unité d'évaluation des données de mesure (12) classe automatiquement les défauts par extraction de caractéristiques à partir des données de mesure et les données collectées sont enregistrées dans une base de données (16) à l'intérieur ou à l'extérieur du dispositif afin de garantir la traçabilité des mesures et de rendre détectables les évolutions temporelles de l'objet à contrôler (8) entre différentes mesures.

17. Utilisation du dispositif selon la revendication 1, dans laquelle des informations relatives aux valeurs cibles et aux caractéristiques spécifiques des objets à contrôler (8) sont préalablement enregistrées dans la base de données (16) afin de garantir une reconnaissance immédiate des formes et une classification des défauts.

18. Utilisation du dispositif selon la revendication 1, dans laquelle les données évaluées sont émises pour un traitement ultérieur afin de transmettre les données de mesure sous forme graphique, imagée, tabulaire ou sous forme de fichier de données de mesure à un dispositif terminal (14) apte à la visualisation ou au stockage.

19. Utilisation du dispositif selon la rrevendication 1, dans laquelle, en cas de disposition d'une option de visualisation, par exemple un écran, sur le dispositif, la commande du dispositif ainsi que la transmission des données de mesure vers le dispositif terminal (14) sont réalisées au moyen d'une transmission de données sans fil, par exemple WLAN.

20. Utilisation du dispositif selon la revendication 1, dans laquelle l'initialisation de l'acquisition des valeurs de mesure est réalisée au moyen du codeur rotatif incrémental du capteur de déplacement (9) afin de garantir la plus grande correspondance possible entre l'emplacement de mesure et la valeur mesurée associée.

21. Utilisation du dispositif selon la revendication 1, dans laquelle des valeurs cibles enregistrées dans la base de données (16), par exemple pour le diamètre, le pas de toronnage, le nombre de fils/torons et le sens d'enroulement, sont rrelevées afin de réaliser une vérification immédiate de l'identité de l'objet à contrôler.

22. Utilisation du dispositif selon la rrevendication 1, afin de réaliser le contrôle d'autres éléments de câble, tels que des cosses, au moyen de procédés d'imagerie optique.
